# EUROPÄISCHE PATENTANMELDUNG

(11) **EP 0 673 679 A1**
(43) Veröffentlichungstag der Anmeldung: **27.09.1995**
(21) Anmeldenummer: 95103907.2
(22) Anmeldetag: 17.03.1995
(51) Int. Cl.: B01L 3/00, C12Q 1/68, B01L 7/00, G01N 1/30

(54) **Vorrichtung zur Bearbeitung von Nukleinsäuren in Präparationen**

(30) Priorität: 22.03.1994 DE 4409705
(71) Anmelder: BOEHRINGER MANNHEIM GMBH, D-68298 Mannheim (DE)
(72) Erfinder: Kandolf, Reinhard, Prof. Dr. med., D-72379 Hechingen (DE)

(57) **Zusammenfassung**

Vorrichtung zur Bearbeitung von Nukleinsäuren in Präparationen, enthaltend einen Träger 1, der mindestens einen flachen transparenten Bereich 2 mit einer Größe, die für das Aufbringen einer Präparation ausreicht, mindestens ein Bauteil 10, welches ein Loch 11 aufweist, und Mittel zum flüssigkeitsdichten Verbinden der Bauteile 1 und 10 enthält sowie damit durchführbare Verfahren.

## Beschreibung

Gegenstand der Erfindung sind Vorrichtungen zur Bearbeitung von Nukleinsäuren in Zellen, beispielsweise in Gewebeschnitten aber auch Cytospins und Chromosomenpräparationen, sowie Verfahren zur Bearbeitung von Nukleinsäuren mit Hilfe dieser Vorrichtungen. Wenn im folgenden von Präparationen die Rede ist, sind also die oben genannten nukleinsäurehaltigen Proben gemeint.

Die Lokalisierung von Nukleinsäuren in Gewebeschnitten ist ein in der Histologie oft verwendetes Verfahren. Die Nukleinsäuren wurden bisher durch relativ unspezifische Färbemethoden sichtbar gemacht. In jüngerer Zeit konnte eine gewisse Spezifität durch Einführung der sogenannten in-situ Hybridisierung erreicht werden. Dazu wird eine für die nachzuweisende Nukleinsäure spezifische, markierte Sonde in Lösung mit einem auf einem Objektträger immobilisierten Gewebeschnitt in Kontakt gebracht. Ein Nachteil dieser in-situ Hybridisierungen war die relativ geringe Empfindlichkeit und der große Zeitbedarf bei der Durchführung des Verfahrens.

Neuerdings wurde der Zeitbedarf durch Einführung der durch Primer-induzierten sequenzspezifischen Markierung (PRINS) verringert. Hierzu wird der immobilisierte Gewebeschnitt mit einem unmarkierten Oligonukleotid, einem oder mehreren markierten Mononukleosidtriphosphaten und einer entsprechenden Polymerase in Kontakt gebracht. Nach Diffusion der Primer und der übrigen Reagenzien, die im Falle kürzerer Nukleinsäuren schneller verläuft, in die Präparation, bilden sich bei Anwesenheit der nachzuweisenden Nukleinsäure markierte Elongate der Primer, die in den Zellen der Präparation zurückbleiben (Biotech Forum Europe, Heft 12/91, S. 752-756 (Hüthig Verlag, Heidelberg)). Ein Nachteil dieser Methode ist eine relativ geringe Sensitivität.

Neuerdings wurden diese Methoden auch mit Amplifikationsmethoden verknüpft. So ist beispielsweise in der EP-A-0 524 808 ein Verfahren zur in-situ PCR in fixierten Zellen unter Einbau von Digoxigenin-11-dUTP beschrieben. Ein Review zu in-situ PCR ist in Virchows Archiv B Cell Pathol. (1993), S. 67-73 gegeben. Zur Reduktion der Verdunstung von Wasser aus dem Reaktionsgemisch über den fixierten Zellen wurde das Gemisch mit Hilfe von Mineralöl gegen die Umgebung abgedichtet.

Aufgabe der vorliegenden Erfindung war es, die bekannten Verfahren und Vorrichtungen zu verbessern, insbesondere die Effizienz und Sensitivität zu steigern und unspezifische Reaktionen zu unterdrücken.

Gegenstand der Erfindung ist daher eine Vorrichtung zur Bearbeitung von Nukleinsäuren in nukleinsäurehaltigen Präparationen, wie Zellen, Gewebeschnitten oder immobilisierten Nukleinsäure, enthaltend
- einen Träger 1, der mindestens einen flachen transparenten Bereich 2 mit einer Größe, die für das Aufbringen der Zellen oder der Nukleinsäuren ausreicht, enthält;
- mindestens ein Bauteil 10, welches ein Loch 11 mit einer oberen (12) und unteren (13) lichten Weite aufweist.
- Mittel zum flüssigkeitsdichten Verbinden der Bauteile 1 und 10.

Ebenfalls Gegenstand der Erfindung ist eine Vorrichtung, die eine Vielzahl der flachen Träger integriert enthält, sowie Verfahren zur Bearbeitung von Nukleinsäuren in Präparationen unter Verwendung der oben genannten Vorrichtungen.

Unter Präparationen werden insbesondere Gewebeschnitte, Einzelzellen- und Nukleinsäurepräparationen verstanden.

Die erfindungsgemäße Vorrichtung ist insbesondere zum Nachweis von Nukleinsäuren in Gewebeschnitten verwendbar, wie sie üblicherweise in der Histologie anfallen. Bezüglich der Herstellung der Gewebeschnitte sind gegenüber den bekannten Methoden keine Änderungen erforderlich. Diese Gewebeschnitte enthalten Nukleinsäuren, deren Anwesenheit oder Abwesenheit mit dem erfindungsgemäßen Verfahren nachgewiesen werden soll. Es sind sowohl qualitative als auch quantitative Nachweise möglich. Es können alle Arten von Nukleinsäuren, wie DNA und RNA, sowohl üblicherweise in Zellen des Gewebeschnittes vorkommende, als auch durch eine Infektion eingedrungene Nukleinsäuren nachgewiesen werden. Insbesondere eignet sich das erfindungsgemäße Verfahren für Untersuchungen von DNA und/oder RNA des Organismus, aus dem der Gewebeschnitt entnommen wurde oder von Fremdnukleinsäure, z. B. von Krankheitserregern.

Die Vorrichtung ist ebenfalls verwendbar für den Nachweis von Nukleinsäuren in Einzelzellen, z. B. in sogenannten Cytospins. Die Einzelzellen können in bekannter Weise an einem Träger immobilisiert werden. Der Nachweis verläuft ansonsten wie der bei Gewebeschnitten.

Die Vorrichtung ist jedoch auch verwendbar für den Nachweis immobilisierter Nukleinsäuren, wie Chromosomen. Die Präparation dieser Nukleinsäuren ist ebenfalls bekannt.

Die erfindungsgemäße Vorrichtung enthält als ein wesentliches Bauteil einen Träger 1, der mindestens einen flachen transparenten Bereich 2 enthält, welcher mindestens so groß ist wie die auf Nukleinsäuren zu untersuchende Fläche. Um diesen transparenten Bereich herum ist der Träger bevorzugt ebenfalls flach, sodaß die für die Behandlung des Probenmaterials nötigen Arbeitsschritte bequem ausgeführt werden können. Flach in Zusammenhang mit Träger 1 bedeutet, daß er mit einem weiteren darauf anzubringenden Bauteil 10 so zusammenpasst, daß die beiden Bauteile flüssigkeitsdicht verbunden werden können. Flach im Zusammenhang mit dem transparenten Bereich 2 bedeutet, daß er an der für die Immobilisierung der Präparation vorgesehenen Stelle keine Erhebungen enthält, die eine ausreichende Immobilisierung der Präparation oder die Fokussierung eines für den Nachweis verwendeten Mikroskops auf das Untersuchungsmaterial behindern.

Bevorzugt besteht der Träger 1 durchgehend aus dem Material des transparenten Bereiches 2. Dieses Material richtet sich nach der Art des Nachweisverfahrens Sollen die Nukleinsäuren beispielsweise durch Einbau einer Markierung nachgewiesen werden, so muß das Material für Licht einer Wellenlänge, mit der die Markierung nachgewiesen wird, durchlässig sein. Als transparente Materialien haben sich insbesondere Glas und Kunststoffe, z. B. Polystyrol bewährt.

Als besonders bevorzugte Träger 1 haben sich die üblicherweise in der Histologie verwendeten Objektträger aus Glas bewährt, wobei diese im Hinblick auf die äußere Form modifiziert werden können.

Ein weiteres wesentliches Bauteil ist Bauteil 10, das in seiner Form auf den Träger 1 abgestimmt ist und eine feste Form aufweist. Es weist ein Loch 11 auf, das sich im wesentlichen senkrecht von der Fläche des transparenten Bereiches 2 erstreckt, wenn das Bauteil 10 so mit dem Träger 1 verbunden ist, daß sich der für die Analyse relevante Bereich der Präparation im wesentlichen innerhalb des Loches befindet. Somit hat das Loch 11 eine obere und untere lichte Weite (12) bzw. (13). Das Loch kann der Form der Präparation oder der Form des Trägers angepaßt sein.

In einer ersten Möglichkeit weist das Bauteil 10 an seiner Unterseite eine Struktur auf, die der Struktur des Trägers 1 um die Präparation herum angepaßt ist, sodaß die Verbindungsstelle zwischen Bauteil 10 und Träger 1 flüssigkeitsdicht verschlossen werden kann. Hierzu sind dann entsprechende Mittel vorgesehen. In diesem Fall ist die untere lichte Weite des Lochs kleiner als die Fläche des Objektträgers. Besonders bevorzugt liegt Bauteil 10 auf Träger 1 flach auf und kann mit Hilfe eines Klebemittels, z. B. doppelseitigen Klebebands oder Rubber Cement aber auch mit Hilfe eines Teflonbands mit dem Träger verbunden und abgedichtet werden. Im Falle eines Teflonbandes als Dichtmittel hat sich die Verwendung einer Metallklammer zum Zusammendrücken der Bauteile 1 und 10 als vorteilhaft erwiesen.

In einer zweiten Möglichkeit ist die untere lichte Weite größer als der äußere Umfang des Trägers 1. In diesem Fall kann das Bauteil 10 aus einem oder mehreren Bauteilen bestehen, die zum einem eine Haltevorrichtung für den Träger 1 ausbilden und zum anderen die Möglichkeit einer Abdichtung des Bauteils 10 gegenüber dem Träger 1 eröffnen. Die Abdichtung kann dann seitlich oder auch unter dem Träger vorgenommen werden.

In einer dritten Ausführungsform hat das Bauteil 10 die ungefähre Form einer Wanne, die zur Aufnahme des Trägers 1 geeignet ist, sodaß sich Mittel zur flüssigkeitsdichten Verbindung des Bauteils mit dem Träger erübrigen.

Bevorzugte Ausführungsformen für Bauteil 10 sind eine flache Platte mit einem Loch oder ein Ring. Das Material des Bauteils 10 muß außer Flüssigkeitsundurchlässigkeit auch inert gegen Nukleinsäuren sowie die zur Bearbeitung der Nukleinsäuren verwendeten Reagenzien, gegebenenfalls auch hitzebeständig sein. Bevorzugt sind jedoch leicht zu bearbeitende Materialien, wie Metalle und Kunststoffe. Als besonders bevorzugtes Material hat sich Teflon herausgestellt. Durch die Dimensionen des Lochs 11 (Fläche, Höhe) wird ein Volumen definiert. Die Fläche des Loches 11 kann sich nach der Größe des zu analysierenden Bereiches der Präparation richten. Die Höhe des Loches ist mindestens so groß, daß genügend Reagenz für die Bearbeitung der Nukleinsäuren zur Verfügung gestellt werden kann, bevorzugt größer als 0.5 mm, besonders bevorzugt zwischen 1 und 10 mm. Die Reagenzlösung sollte bevorzugt mindestens in einer Höhe von 0.5 mm über der Präparation stehen.

Es ist prinzipiell möglich, eine Ausführungsform zu wählen, bei der die gesamte Fläche des Trägers vom Loch des Bauteils 10 überspannt wird. Das Volumen ist bei üblichen Präparationen auf einem Objektträger für den Fall, daß Bauteil 10 rund ist und eine Präparation gerade umfaßt, bevorzugt mindestens 50 µl groß, besonders bevorzugt mehr als 100 µl groß. Als praktische Untergrenze bei Überspannen der Gesamtfläche eines Objektträgers hat sich ca. 1 ml herausgestellt.

Das Loch 11 des Bauteils 10 hat auf der dem Träger 1 abgewandten Seite eine obere lichte Weite 12. Diese ist im allgemeinen nicht sehr stark unterschiedlich von der unteren lichten Weite. Oft werden die obere und die untere lichte Weite gleich sein.

Ein vorteilhafterweise vorhandenes wesentliches Bauteil der erfindungsgemäßen Vorrichtung ist eine Abdeckung 20, welche mindestens die obere lichte Weite 12 des Loches 11 abdeckt. Sie ist dem oberen Rand des Bauteils 10 in der Form angepaßt, so daß das Loch 11 damit verschlossen werden kann. Sinn der Abdeckung ist die Verringerung des Flüssigkeitsverlustes (insbesondere bei Erwärmung der Reaktionsmischung) aus dem Reaktionsraum, da eine Verdunstung von Wasser eine Änderung der Konzentration der Komponente in der Reaktionsmischung zur Folge hätte. Als Materialien haben sich Metalle, Glas oder Kunststoffe bewährt, wobei aus praktischen Gründen (im Fall einer Erwärmung der Reaktionsmischung) die Möglichkeit eines Druckausgleiches bestehen sollte.

Wie oben beschrieben, sind gegebenenfalls Mittel zum flüssigkeitsdichten Verbinden des Trägers mit dem Bauteil 10 nötig. Bevorzugte Mittel sind oben beschrieben. Besonders bevorzugt sind Mittel, die zwar während der Reaktion beständig sind, jedoch nach Abschluß der Reaktion zum Nachweis der Nukleinsäuren auf dem Träger auf einfache Weise, z.B. durch Abziehen mit oder ohne den Bauteil 10, entfernt werden können. Die Verbindung des Bauteils 10 mit der Abdeckung 20 soll bevorzugt ebenfalls lösbar gestaltet sein. Daher haben sich hierfür besonders physikalische Verschlußmittel bewährt, z. B. Klammern oder Federn, welche die Abdeckung auf das Bauteil drücken.

Für den Fall einer Abdichtung des Bauteils 10 mit dem Träger 1 von unten kann die Verbindung auch physikalisch, z. B. über einen Schliff stattfinden. Diese Abdichtung kann durch einen Stempel, der auf den Träger im Bereich der Haltevorrichtung drückt, verstärkt werden.

Dieser feste Verschluß durch die Abdeckung macht die Verwendung von Mineralöl in der erfindungsgemäßen Vorrichtung überflüssig. Durch den Austausch des Mineralöls durch eine feste Abdeckung wird es möglich, der Vorrichtung durch einfaches Entfernen der Abdeckung selbst während des Nachweisverfahrens gegebenenfalls notwendige Reagenzien zuzugeben. Dadurch ist es möglich, auch mehrstufige Nachweisverfahren auf einfache Weise in der Vorrichtung durchzuführen.

Ebenfalls Gegenstand der Erfindung ist eine Vorrichtung zur Bearbeitung von Nukleinsäuren in mehreren Präprationen enthaltend
- eine Trägerplatte 30,
- eine Vielzahl von Trägern 1, die jeweils mindestens einen flachen transparenten Bereich 2 mit einer Größe, die für das Aufbringen eines Gewebeschnittes ausreicht, aufweisen, und
- ein flaches Bauteil 40, welches mehrere Löcher von der Form der transparenten Bereiche 2 enthält.

Gegebenenfalls enthält sie auch Mittel zum flüssigkeitsdichten Verbinden der Träger 1 mit dem Bauteil 40.

Diese Vorrichtung unterscheidet sich dadurch von der oben genannten erfindungsgemäßen Vorrichtung, daß zur Durchführung des Nachweisverfahrens parallel in mehreren Präparationen die Bauteile 10 und 20 für mehrere Präparationen in einem Bauteil zusammengefaßt sind. Die flachen Träger 1 bleiben hierbei bevorzugt voneinander getrennt, werden jedoch auf einer Trägerplatte, die eine Vielzahl dieser flachen Träger enthält, an vorbestimmten Orten festgehalten. Die bevorzugte Anzahl von flachen Trägern, die auf einer Trägerplatte angeordnet werden können, beträgt 2 bis 20, besonders bevorzugt 4 bis 16. Zum Festhalten der flachen Träger weist die Trägerplatte bevorzugt Ausnehmungen in Form der flachen Träger auf. Ebenfalls bevorzugt ist die Trägerplatte 30 aus einem gut wärmeleitenden Material, besonders bevorzugt einem Metall aufgebaut. Sie kann für mehrere Bestimmungen hintereinander ohne substantielle Reinigung verwendet werden, da sie mit den Reaktionsgemischen in den einzelnen Vorrichtungen nicht in Kontakt kommt. Die Deckplatte sollte ein Einmalartikel oder leicht zu reinigen sein.

Ebenfalls Gegenstand der Erfindung ist ein Verfahren zur Bearbeitung von Nukleinsäuren in Präparationen, bei dem die Nukleinsäuren innerhalb der Präparation die auf einer Vorrichtung, wie oben geschildert, immobilisiert ist, in der Vorrichtung bearbeitet und gewünschtenfalls anschließend auf dem Träger nachgewiesen werden. Das erfindungsgemäße Verfahren umfaßt daher im wesentlichen folgende Schritte:
- Fixierung der Präparation auf dem flachen Bereich 2 des Trägers 1;
- Verbinden des Bauteils 10 mit Träger 1;
- Einfüllen von für die Bearbeitung erforderlichen Reagenzien;
- Gegebenenfalls Abdeckung der Reaktionsmischung,vorzugsweise durch eine Abdeckung 20 oder eine Mineralölschicht;
- Inkubation der Präparation in der Reaktionsmischung unter den für die Bearbeitung geeigneten Bedingungen, (z. B. cDNA-Synthese).
- Gegebenenfalls zyklische Erhöhung bzw. Erniedrigung der Temperatur der Reaktionsmischung, sofern eine Amplifikation der vorhandenen Nukleinsäuren (z. B. PCR) stattfindet.
- Gegebenenfalls Entfernung der Abdeckung 20;
- Entfernung des Reaktionsüberstandes aus der Vorrichtung, ggf. mit Waschen.

Für den Fall des Nachweises der Nukleinsäuren in der Vorrichtung schließen sich bevorzugt folgende Schritte an:
- Einfüllen von Reagenzien, die für die Detektion eventuell gebildeter Nukleinsäuren in der Präparationen geeignet sind, beispielsweise markierte Nachweissonden, oder Reagentien zum Nachweis markierter Nukleinsäuren;
- Gewünschtenfalls erneuter Verschluß der Vorrichtung;
- Erforderlichenfalls erneutes Öffnen der Vorrichtung und Entfernen der Reagenzlösung und Zugabe weiterer Nachweisreagenzien; und
- Nachweis eines für die Markierung charakteristischen Signales, vorzugsweise nach Entfernung des Bauteils 10, beispielsweise unter einem Mikroskop.

Für die gleichzeitige Untersuchung mehrerer Gewebeschnitte werden die Träger 1 mit den darauf fixierten Gewebeschnitten in die dafür vorgesehenen Ausnehmungen eingelegt, das Bauteil 40 aufgelegt und flüssigkeitsdicht mit den Trägern 1 verbunden. Nach Zugabe der Reagenzlösungen in die gebildeten Räume wird die Abdeckplatte 50 aufgelegt und mit dem Bauteil 40 verbunden. Die übrigen Schritte verlaufen analog zum Einzeltest wie oben geschildert.

Die erfindungsgemäße Vorrichtung ist besonders geeignet für die Durchführung von Nukleinsäureamplifikationsverfahren, die in Thermocyclen verlaufen. Solche Amplifikationsverfahren sind beispielsweise beschrieben in der EP-A-0 201 184 (Polymerase Chain Reaction) und EP-A-20 308 (Ligase Chain Reaction) oder WO 90/01063 (Repair Chain Reaction). Während alle für diese Amplifikationsverfahren dort beschriebenen wesentlichen Bedingungen eingehalten werden müssen, hat es sich für die erfindungsgemäße Bearbeitung in Präparationen herausgestellt, daß für eine normale Präparation der Flüssigkeitsspiegel der Reaktionsmischung mindestens 0,5 mm über der Präparation steht. Die Handhabung dafür erforderlicher Volumina war auf den bisher verwendeten Vorrichtungen für solche Präparationen praktisch nicht möglich. Die Amplifikationsrate in den verschiedenen Präparationen mittels erfindungsgemäßer Vorrichtung liegt deutlich höher, als die bisher erreichbaren Raten; gezeigt werden konnte eine Erhöhung der Amplifikationsrate auf das 1000fache und mehr. Zur Durchführung der Thermocyclen kann Träger 1 auf die Metallplatte eines Thermocyclers gelegt werden. Dadurch kann die Temperatur in der Reaktionsmischung genau gesteuert werden. Ein Vorteil der erfindungsgemäßen Vorrichtung ist, daß auch Temperaturfühler in die Reaktionsmischung eingetaucht werden können.

In einer bevorzugten Ausführungsform des Verfahrens ist entweder eines der in der Amplifikation eingesetzten Mononukleosidtriphosphate oder eines der Oligonukleotide nachweisbar markiert. Besonders bevorzugte nachweisbare Markierungen sind kleine Moleküle, wie Haptene, die über eine spezifische Wechselwirkung mit einer nachweisbaren Verbindung, z. B. fluorometisch oder colorimetrisch nachgewiesen werden können.

Vorteile der erfindungsgemäßen Vorrichtung und des Verfahrens sind hohe Sensitivität und Effizienz des Verfahrens. Außerdem ist das Verfahren leicht automatisierbar.

Die erfindungsgemäße Vorrichtung kann auch zur Durchführung anderer Bearbeitungsverfahren von Nukleinsäurehaltigen Präparationen verwendet werden, z. B. Charakterisierung der in der Präparation enthaltenen Nukleinsäuren. Nach Lyse der Zellen können die Nukleinsäuren mit dem Überstand aus der Vorrichtung entnommen und weiteren Reaktionen zugänglich gemacht werden, z.B. einer Clonierung oder Sequenzierung.

FIG 1 zeigt in Explosionsdarstellung den Aufbau einer bevorzugten erfindungsgemäßen Vorrichtung mit einem Gewebeschnitt G für eine Untersuchung.

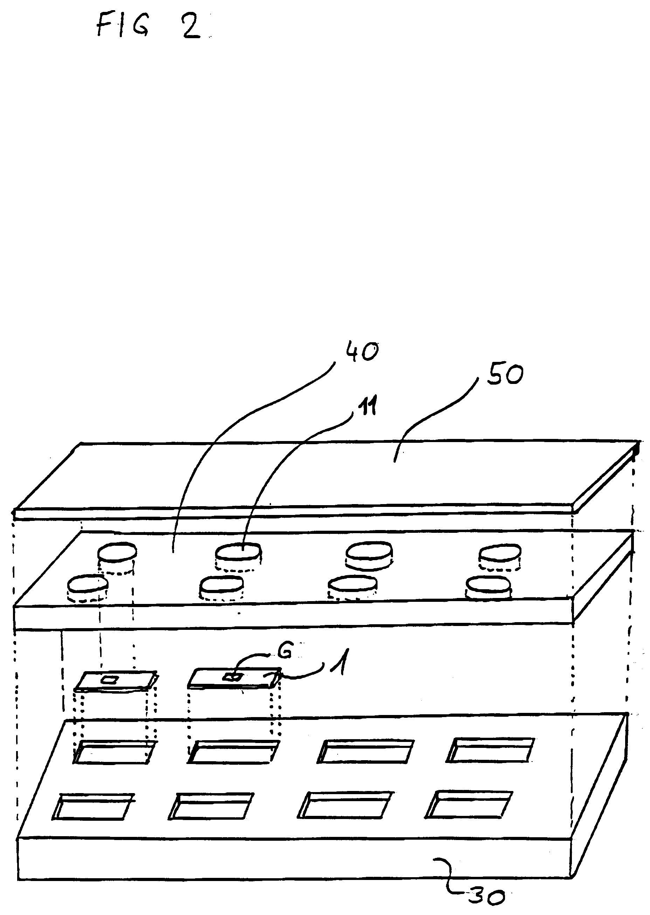

FIG 2 zeigt den Aufbau für mehrere Untersuchungen.

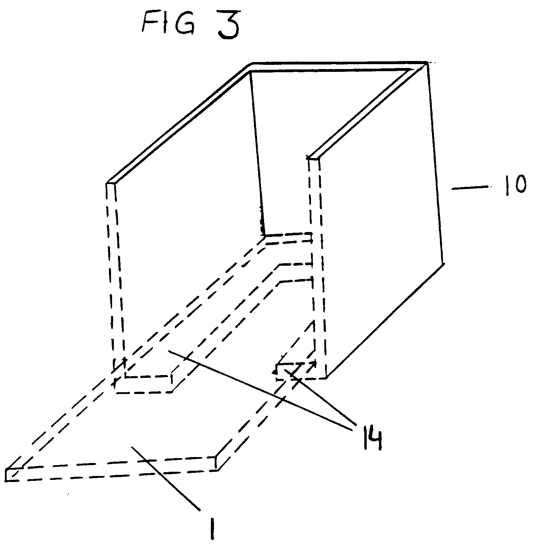

FIG 3 zeigt eine Ausführungsform, in der die lichte Weite des Bauteils 10 größer ist als der Träger 1 im Querschnitt mit angedeutetem Träger 1. Das Bauteil 10 enthält Haltevorrichtungen 14 für den Träger 1. Die Haltevorrichtungen verlaufen unter dem gesamten Träger 1 entlang. Sofern Bauteil 10 (entweder über die Seiten oder die Haltevorrichtungen) nicht schon dicht gegenüber Träger 1 ist, können oben genannte Mittel zum Verbinden vorgesehen sein. Ein Vorteil dieser Ausführungsform ist, daß der Träger 1 nach Durchführung der Reaktion wieder auf einfache Weise durch Druck von unten aus dem Bauteil 10 zu entnehmen ist.

### Bezugszeichenliste

- 1: Träger
- 2: transparenter Bereich
- G: Gewebeschnitt / Präparation
- 10: Bauteil mit Loch (Durchbohrung)
- 11: Loch
- 12: lichte obere Weite
- 13: lichte untere Weite
- 14: Haltevorrichtung für Träger 1
- 20: Abdeckung
- 30: Trägerplatte
- 40: Bauteil mit mehreren Löchern
- 50: Deckplatte

Die folgenden Beispiele sollen die Erfindung näher erläutern:

### Beispiel 1

### Gewebeprozessierung

Gewebeproben werden für zumindet 4 h bei 4°C in 1,5 % Paraformaldehyd/1,5 % Glutardialdehyd/0,1 M Natriumposphat (pH 7,2) fixiert und nach Dehydrierung mit Ethanol entsprechend Standardtechniken in Paraffin (Paraplast) eingebettet. Zumindest vier micrometer dicke Paraffin-Gewebeschnitte werden auf gereinigte und beschichtete Glasobjektträger aufgebracht wie für die in-situ Hybridisierung beschrieben (Klingel et al. 1992, Hohenadl et al. 1991). Entsprechend dem Vorgehen bei Gewebeproben werden native Zellen nach Aufbringung auf Glasobjektträger für zumindest 20 min. bei 4°C fixiert und dehydriert (Hohenadl et al. 1991). Vor Dru chführung der in-situ PCR werden die Gewebeschnitte entsprechend Standardtechniken entwachst, mit Proteinase K (1 µg/ml) vorbehandelt (Klingel et al. 1992), gefolgt von einer Inkubation für 15 min bei 65°C in 95 % Formamid/0,1xSSC und einer Waschung für 5 min. bei 4°C in 0,1xSSC. Die in-situ cDNA Synthese sowie die in-situ Amplifikation erfolgt in zumindest 7,5 mm weiten Zylindern, die mit Hilfe einer doppelklebenden Folie oder einer Gummidichtung um die Gewebeschnitt auf den Glasobjektträger aufgebracht werden. Zur Vermeidung einer Evaporation der Reaktionslösungen werden die Zylinder mit einem adhäsiven Plastikfilm bedeckt, der einen Druckausgleich ermöglicht.

### Beispiel 2

### In-situ Transkription

Die cDNA Synthese erfolgt unter Verwendung synthetischer komplementärer DNA-Oligonukleotide (50 µg/ml) und reverser Transkriptase (2000 U/ml) für 40 min. bei 42°C in zumindest 50 µl 100 mM Tris-HCl (pH 8,3), 5 mM MgCl₂, 100 mM KCl, 1 mM jeweils von dATP, dCTP, dGTP sowie TTP, 0,4 mM DDT und RNasin (500 U/ml). Nach erfolgter cDNA Synthese wird die Lösung aus den Reaktionszylindern abgesaugt und nach einer Waschung des Gewebes mit 0,1xSSC durch die in-situ PCR Reaktionslösung ersetzt.

### Beispiel 3

### In-situ Polymerase-Kettenreaktion (PCR)

Die in-situ PCR erfolgt in zumindest 100 µl einer Reaktionslösung bestehend aus 10 mM Tris-HCl (pH 8,3), 50 mM KCl, 1,5 mM MgCl₂, 0,001 % Gelatine, 1 mM jeweils von dATP, dCTP sowie dGTP, 0,75 mM TTP, 0,25 mM bio-16-dUTP, 200 pM Primer 1 und 2, 5 U Taq-Polymerase. Die PCR Konditionen unter Verwendung eines DNA-Thermo-Cyclers sind zumindest 15 Cyklen für 1 min. bei 94°C, bis zu 3 min. bei 55°C und bis zu 3 min. bei 72°C. Nach Entfernung der PCR Reaktionslösung und der Reaktionszylinder erfolgt eine Inkubation der Glasobjektträger bei 20°C für 30 min. in 2xSSC gefolgt von 15 min. bei 20°C in 0,01xSSC.

### Beispiel 4

### Nachweis der in-situ PCR-Produkte durch Immunhistochemie

Der Nachweis biotinylierter Amplifikationsprodukte erfolgt durch Inkubation der Gewebeschnitte oder Zellpräparationen für 4 h bei 4°C mit Anti-Biotin Kaninchen-Antikörpern (Enzo, New York, NY) in einer 1:100 Verdünnung in PBS⁻/1% BSA. Anschließend erfolgt eine Inkubation für 1 h bei 20°C mit Colloidalgold-konjugierten Ziege anti-Hase IgG Antikörpern. Die Goldpartikel (5 nm) werden durch Silberverstärkung (IntenSE^{TH}M, Amersham Corp.) mikroskopisch sichtbar gemacht.

### Beispiel 5

### Untersuchungen zur Sensitivität der in-situ PCR am Beispiel der chronischen Myokarditis

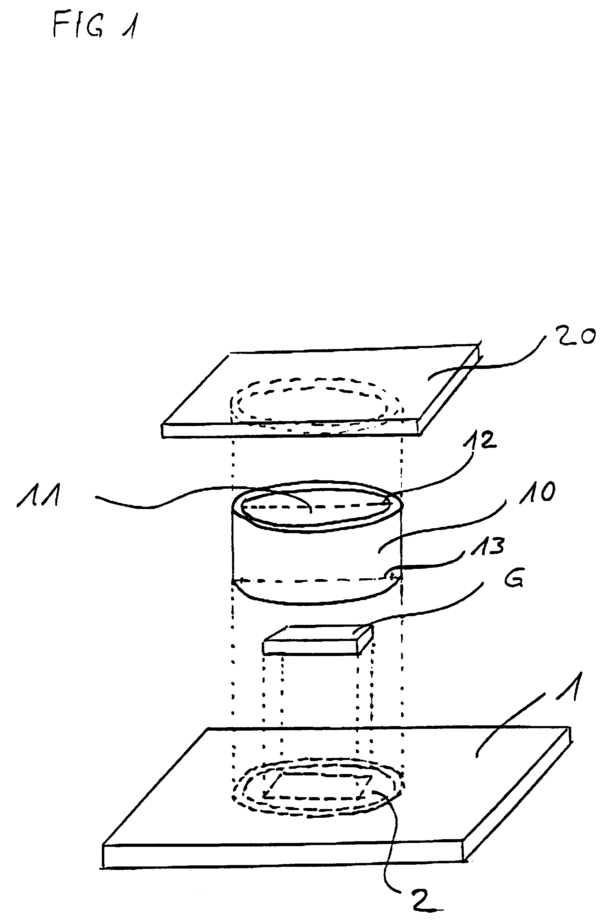

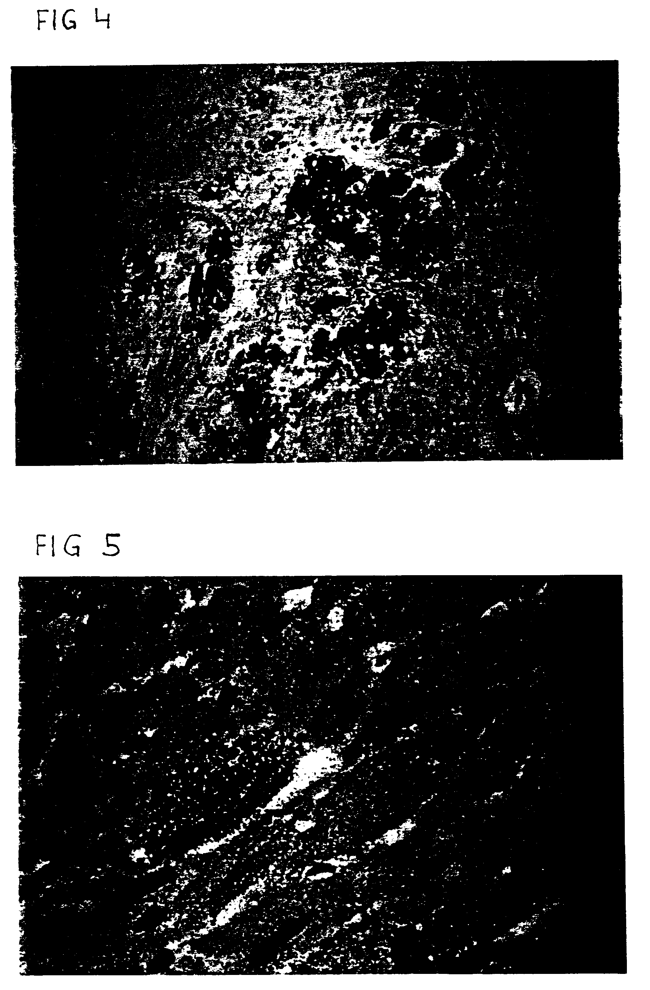

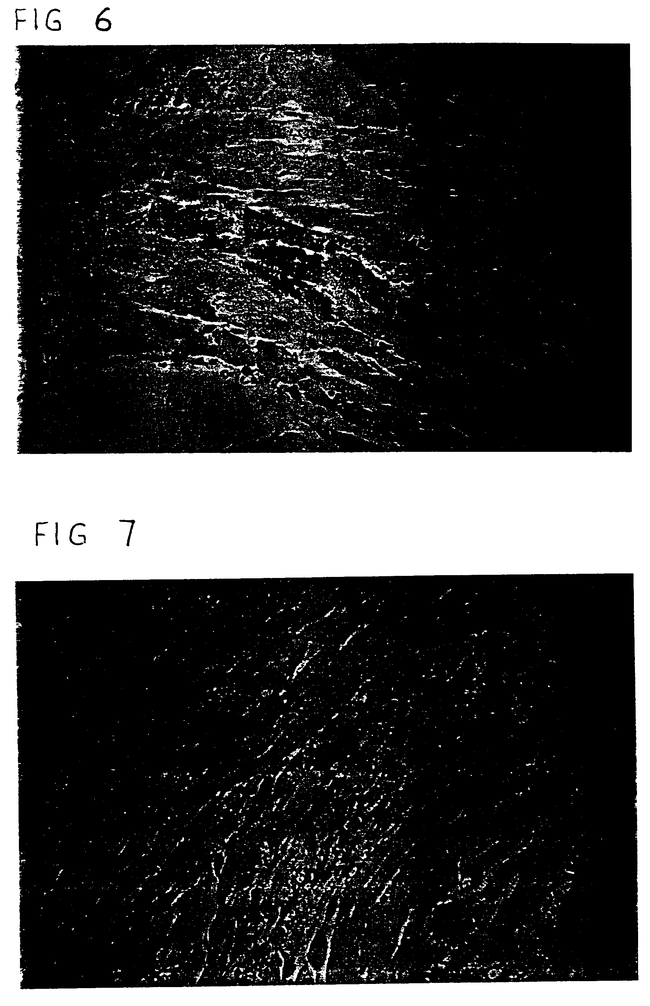

Ausgehend von in-situ Hybridisierungsbefunden zum Nachweis einer perisitierenden enteroviralen Infektion des Herzmuskels bei Mensch und Maus (Kandolf and Hofschneider 1989, Klingel et al. 1992) wurden vergleichend persistierende Infektionsmuster des Herzens durch in-situ PCR wie oben beschrieben sowie durch konventionelle in-situ Hybridisierung untersucht. Zum Nachweis der persistierenden Infektion durch in-situ PCR wurden die folgenden Primer eingesetzt: Primer 1 ist identisch mit den Nukleotiden 63 bis 82 der publizierten Sequenz von Coxsackievirus B3 (CVB3) (Klump et al., 1990): 5'-CGG TAC CCT TGT GCG CCT GT-3', Primer 2 ist komplementär zu den Nukleotiden 395 bis 376 von CVB3 (Klump et al. 1990): 5'-CAG GCC GCC AAC GCA GCC-3' und wird auch für die in-situ Transkription eingesetzt. FIG 4 zeigt in typischer Weise den in-sitz PCR Nachweis eines persistierenden enteroviralen Infektionsmusters des Herzens. FIG 5 zeigt sodann in eindrucksvoller Weise, daß mit Hilfe der oben beschriebenen in-situ PCR Technik die erhaltenen Amplifikationsprodukte exakt infizierten Muskelfasern zugeordnet werden können. Wichtig ist festzuhalten, daS die Stärke der Silber-Goldverstärkung der in-situ Amplifikationsprodukte persistent infizierter Zellen (FIG 4) der Intensität der Färbung akut infizierter Zellen nach konventioneller in-situ Hybridisierung entspricht. FIG 6 zeigt einen entsprechenden Nachweis akut infizierter Herzzellen unter Verwendung einer biotinylierten Hybridisierungssonde durch konventionelle in-situ Hybridisierung. Wichtig ist ferner festzuhalten, daß es mit konventioneller in-situ Hybridisierung unter Verwendung biotinylierter Hybridisierungssonden im Gegensatz zur in-situ PCR nicht gelingt, persistierende Infektionsmuster nachzuweisen. FIG 7 zeigt einen entsprechenden negativen Befund mit fehlendem Nachweis der Viruspersistenz durch konventionelle nicht radioaktive in-situ Hybridisierung. Für die Berechnung der Sensitivität der in-situ Hybridisierung können auf der Grundlage radioaktiver in-situ Hybridisierungsstudien (Kandolf et al. 87, Kandolf 1988) folgende Aussagen gemacht werden.
(i) Akut infizierte Myozyten replizieren durchschnittlich 50.000 virale Kopien pro Zelle.
(ii) Der Vergleich persistierender Infektionsmuster dargestellt durch in-situ PCR (Abb. 1) bzw. radioaktive in-situ Hybridisierung (Klingel et al. 1992) zeigt, daß nach Durchführung der in-situ PCR signifikant mehr Zellen als infiziert nachweisbar sind als nach Durchführung der konventionellen in-situ Hybridisierung mit ³⁵S-markierten Hybridisierungssonden.

Da gezeigt wurde, daß bei radioaktiver in-situ Hybridisierung nach 14-tägiger Exposition die Nachweisgrenze bei 20 viralen Genomen liegt (Kandolf et al. 1987), kann gefolgert werden, daß die erreichte Amplifikationsrate in der in-situ PCR zumindest einem Faktor von 2.500 (50.000 dividiert durch 20) entspricht, da die Intensität der immunhistochemischen Färbung persistent infizierter Zellen derjenigen akut infizierter Zellen entspricht (vgl. FIG 4 mit FIG 6). Da, wie bereits ausgeführt, durch in-situ PCR zudem signifikant mehr infizierte Zellen während der persistierenden Infektion im Vergleich zur radioaktiven in-situ Hybridisierung nachweisbar sind, kann zumindest gefolgert werden, daß die Sensitivität der entwickelten Methode größer ist als diejenige der radioaktiven in-situ Hybridisierung. Setzt man die Nachweisgrenze vorsichtig mit zumindest 10 viralen Kopien an, kann der Amplifikationsfaktor nochmals verdoppelt werden, sodaß eine 5.000fache Amplifikation für die entwickelte in-situ PCR erreicht wird. Damit liegt die Sensitivität der beschriebenen Methode zumindest um einen Faktor von 50 höher als bislang in der Literatur für die in-situ PCR beschrieben (Komminoth and Long 1993).

### Referenzen

Hohenadl C, Klingel K, Mertsching J, Hofschneider PH, and Kandolf R (1991) Strand-specific dtection of enteroviral RNA in myocardial tissue by in-situ hybridization. Molecular and Cellular Probes 5, 11-20
Kandolf R (1988) The impact of recombinant DNA technology on the study of enterovirus heart disease. In: Coxsackieviruses - A General Update. M Bendinelli, H Friedman, eds, Plenum Press, New York, pp. 293-318
Kandolf R, Ameis D, Kirschner P, Canu A, and Hofschneider PH (1987) In-situ detection of enteroviral genomes in myocardial cells by nucleic acid hybridization: An approach to the diagnosis of viral heart disease. Proc. Natl. Acad. Sci. USA 84, 6272-6276
Kandolf R, Hofschneider PH (1989) Viral Heart Disease. Springer Semin Immunopathol 11, 1-13
Klingel K, Hohenadl C, Canu A, Seemann M, Mall G, and Kandolf R (1992) Ongoing enterovirus-induced myocarditis is associated with persistent heart muscle infection: Quantitative analysis of virus replication, tissue damage and inflammation. Proc. Natl. Acad. Sci. USA 89, 314-318
Klump WM, Bergmann I, Müller BC, Ameis D and Kandolf R (1990) Complete nucleotide sequence of infectious coxsackievirus B3 cDNA: Two initial 5' uridine residues are regained during plus-strand RNA synthesis. J. Virol 64, 1573-1583
Komminoth P and Long H (1993) In-situ polymerase chain reaction. Virchows Archiv B Cell Pathol 64, 67-73.

## Patentansprüche

1. Vorrichtung zum Bearbeiten von Nukleinsäuren in nukleinsäurehaltigen Präparationen enthaltend
- einen Träger 1, der mindestens einen flachen transparenten Bereich 2 mit einer Größe, die für das Aufbringen einer Präparation ausreicht, enthält;
- mindestens ein Bauteil 10, welches ein Loch 11 mit einer oberen (12) und unteren (13) lichten Weite aufweist.

2. Vorrichtung nach Anspruch 1, dadurch gekennzeichnet, daß sie außerdem eine Abdeckung 20 enthält, welche mindestens die obere lichte Weite 12 des Loches 11 abdeckt.

3. Vorrichtung nach Anspruch 1, dadurch gekennzeichnet, daß der Träger 1 ein Objektträger ist.

4. Vorrichtung nach Anspruch 1, dadurch gekennzeichnet, daß sie Mittel zum flüssigkeitsdichten Verbinden der Bauteile 1 und 10 enthält.

5. Vorrichtung nach Anspruch 1, dadurch gekennzeichnet, daß der gebildete Raum eine Höhe von mindestens 0.5 mm aufweist.

6. Vorrichtung zur Bearbeitung von Nukleinsäuren in Präparationen enthaltend
- eine Trägerplatte 30,
- eine Vielzahl von Trägern 1, die jeweils mindestens einen flachen transparenten Bereich 2 mit einer Größe, die für das Aufbringen einer Präparation ausreicht, aufweisen, und
- ein flaches Bauteil 40, welches mehrere Löcher von der Form der transparenten Bereiche 2 enthält.

7. Vorrichtung nach Anspruch 9, dadurch gekennzeichnet, daß die Trägerplatte 30 aus einem gut wärmeleitenden Material aufgebaut ist.

8. Verfahren zur Vermehrung von Nukleinsäuren in Präparationen dadurch gekennzeichnet, daß die Nukleinsäuren in einer auf dem flachen Träger einer Vorrichtung nach Anspruch 1 fixierten Präparation amplifiziert werden.

9. Verfahren nach Anspruch 5, dadurch gekennzeichnet, daß die Amplifikation in Gegenwart nachweisbar markierter Nukleotide vorgenommen wird.

10. Verfahren zum Nachweis von Nukleinsäuren in Präparationen, dadurch gekennzeichnet, daS die Nukleinsäuren in eine auf dem flachen Träger einer Vorrichtung nach Anspruch 1 fixierten Präparation amplifiziert und anschließend nachgewiesen werden.

11. Verfahren nach Anspruch 10, dadurch gekennzeichnet, daS zwischen Nachweis und Amplifikation das Bauteil 10 entfernt wird.
